# EUROPEAN PATENT APPLICATION

(11) **EP 4 796 170 A1**
(43) Date of publication of application: **26.08.2026**
(21) Application number: 25159569.0
(22) Date of filing: 24.02.2025
(51) Int. Cl.: A61N 5/10

(54) **METHODS AND SYSTEM FOR DETERMINING A 3D DOSE DISTRIBUTION IN WATER FROM BEAM PROFILES FOR RADIOTHERAPY PURPOSES**

(71) Applicant: PTW - Freiburg Physikalisch-Technische Werkstätten Dr. Pychlau GmbH, 79115 Freiburg (DE)
(72) Inventor: VON VANGEROW, Johannes, 79108 Freiburg (DE); SCHWAMM, Frank, 79111 Freiburg (DE); BLAUTH, Simon, 79111 Freiburg (DE); KRANZER, Rafael Demian, 79102 Freiburg (DE); WÜRFEL, Jan Ulrich, 79102 Freiburg (DE)
(74) Representative: Mertzlufft-Paufler, Cornelius

(57) **Abstract**

The present disclosure relates to methods for training and using a neural network for calculating a 3D dose distribution in water from measured beam profiles for use in radiotherapy. The present disclosure also relates to a detector system (300) comprising a profile detector (302) and a computing system (304) configured to perform the methods.

## Description

### Field

The present disclosure relates to the calculation of a 3D dose distribution in water from beam profiles for use in the field of radiotherapy.

### Background

A major goal of radiation therapy (RT) is to treat cancers in human tissue. During the treatment, patients are positioned in the vicinity of a radiation source such that the tumor is irradiated with high energy particles, leading to the destruction of cancerous tissue. The irradiation is carried out such that the hazardous impact to healthy tissue is minimized.

The radiation source is, for instance, a linear accelerator (LINAC), whereby a target, such as a tumor, is irradiated by high-energy particles. LINACs are highly complex medical devices, with the primary function to create well defined and controlled dose distributions within the patient. In order to be able to adapt to different treatments, LINACs offer different treatment beams, characterized by the term radiation quality. The radiation quality defines the type of particles (typically photons or electrons) and the spectral energy distribution of the beam, as well as whether a flattening filter is involved to create a flat beam profile. A beam profile defines the dose distribution of the emitted LINAC radiation in any direction perpendicular to the beam propagation direction. State-of-the-art LINAC systems involve a rotatable treatment head and a multileaf collimator, allowing to change the treatment beam propagation direction and its lateral shape in real-time, thereby enabling the creation of tailored dose distributions in the patient treatment volume.

When a LINAC is installed, the machine needs to be commissioned prior to patient treatment. Commissioning involves, among other tests, detailed measurements using a water phantom which can take several days. For each beam quality of the LINAC (this can be up to 6 photon energies or more and as many electron energies), beam profiles in different depths and percentage depth dose (PDD) curves have to be measured for different field sizes in order to collect the beam data. During the operational life of the LINAC, some of these checks are frequently repeated in time intervals defined according to standards or quality assurance plans of a clinic to ensure safe, precise and reliable patient treatment. LINAC commissioning data is used to create a beam model which is used by the treatment planning system (TPS).

A Treatment Planning System in radiation therapy is a specialized software platform used to design and optimize radiation treatments for cancer patients. Its primary purpose is to ensure that the prescribed dose of radiation is accurately delivered to the tumor while minimizing exposure to surrounding healthy tissues. Prior to patient treatment, the dose distribution within the patient resulting from the LINAC radiation is calculated by the TPS.

As a first step prior to commissioning, acceptance testing takes place which includes basic safety checks, energy verification, dose rate measurements and mechanical functionality. After acceptance testing, the first step in the actual commissioning process is given by beam data collection and characterization, where the objective is to precisely characterize and calibrate the different radiation qualities provided by the LINAC. For each radiation quality, rectangular and particularly quadratic field distributions ranging from 40 x 40 cm² to 2 x 2 cm² are created by the collimation system of the LINAC. The corresponding dose distribution is measured with a water phantom, mimicking human tissue, where the water surface is typically positioned in 90 - 100 cm distance from the radiation source. The water phantom system incorporates a detector, typically an ionization chamber or a solid-state detector connected to an electrometer. This chamber can be moved in three dimensions by a translation stage system. The detector is moved through the phantom such that lateral dose profiles and the dose profile in beam propagation direction, termed as percentage depth dose curve (PDD), are measured. Often, more than one detector is used, depending on the context of the measurements. By using a calibrated detector such as an ionization chamber or a solid-state detector, the dose in water in the unit gray (Gy) can be determined. The provided dose value can be used to calibrate the monitor chamber of the LINAC. A monitor chamber is an ionization chamber typically positioned in the treatment head of the LINAC that measures the output of the LINAC in monitor units (MU) in real time.

In a third step, the collected beam data is used to create a mathematical model within the treatment planning system (TPS), enabling accurate dose calculations. The TPS manual provides a detailed list of beam parameters, in particular PDDs and beam profiles, which have to be provided to enable the TPS to calculate its beam model. The beam model is later used by the TPS to calculate dose distributions within the patient.

The present disclosure provides systems and methods as efficient, space-saving and time-saving alternatives for such LINAC commissioning.

### Summary of the Invention

A goal of the invention is to create an efficient, space- and time-saving saving alternative to elaborate 3D water tank scans needed for LINAC commissioning. By a trained neural network (NN), full 3D dose distributions in water are calculated from beam profiles measured with a profile detector in one or only a few (for example N = 1 - 3) depths, using both detector-intrinsic and external buildup material.

A profile detector or array detector herein is a device consisting of multiple small radiation detectors, such as ionization chambers or solid state detectors diodes, arranged in a well-defined, fixed pattern. The pattern is realized by at least two radiation detectors separated from each other in at least one dimension different from the beam propagation direction. Both profile and array detectors can be suitable detection systems in the scope of the present invention.

To give examples, possible implementations are 2D array detector grids with a well-defined spacing in two dimensions or beam profile arrangements, where detectors are positioned in a star-shaped pattern (4 or 2 profile-axes separated by 45° or 90° from each other, lying in a plane perpendicular to the beam propagation direction). As another example, an Electronic Portal Imaging Device (EPID) may be used as a profile detector within the understanding and scope of the present invention. In addition, a detector such as an ionization chamber or a solid-state detector mounted onto a translation stage movable in up to 3 dimensions can be understood as a suitable detection system in the scope of the present invention.

Besides the material necessary to provide the detection functionality, radiation detectors are surrounded by material such as polystyrene, having dosimetric properties and/or radiological properties similar to water, termed as water-equivalent material. By using profile detectors, the dose distribution in water-like material can be measured, simplifying the measurement as compared to a water phantom measurement.

Profile detectors are typically used in LINAC quality assurance for frequent constancy checks and measurement tasks, where a full water phantom is not needed. By using profile detectors, beam profiles can be measured in a time efficient manner, allowing quick maintenance checks of LINAC beams. Up to now, profile (or array) detectors are not used as a sole detection system for beam data collection and characterization during commissioning. As an additional application, in combination with a phantom consisting of solid, water-equivalent material, in particular array detectors can be used to verify patient treatment plans. The purpose is to check whether the dose distribution planned by the TPS is really delivered by the LINAC.

A first aspect of the invention relates to a method for training a neural network for calculating a 3D dose distribution in water, in particular for use in radiotherapy, the method comprising:
- Determining at least one beam profile, in particular in a water-equivalent depth of at least 3 mm, in particular within a medium comprising at least one solid component, in particular for predetermined irradiation parameters, in particular using a profile detector,
- Calculating a 3D dose distribution in water, in particular for the predetermined irradiation parameters, based on the recorded at least one beam profile,
- Training a neural network for calculating the 3D dose distribution in water using the determined at least one beam profile and the calculated 3D dose distribution in water.

In other words, the invention involves training a neural network (NN) to calculate a 3D dose distribution in water, comprising: Recording at least one beam profile using a profile detector for given irradiation parameters; Calculating a 3D dose distribution in water based on the measured at least one beam profile; Train a NN with beam profile data sets and corresponding 3D dose distributions in water.

In an embodiment, the at least one beam profile is determined in a water-equivalent depth of at least 3 mm in a medium comprising at least one solid component. The at least one component and/or the medium may preferably be solid and preferably exhibit water-equivalent radiological properties. For example, the medium may comprise one component which may be polystyrene. However, the surrounding medium can also be a metal, a gas or a liquid, except water.

In an embodiment, the medium comprises more than one component. For example, the medium providing a water-equivalent depth of at least 3 mm may be a solid containment filled with a liquid. In another example, the medium providing a water-equivalent depth of at least 3 mm may be a solid containment of a material, filled with a gas, e.g. air or an inert gas. In another example, the medium providing a water-equivalent depth of at least 3 mm may comprise layers of same or different solid material sheets. For example, the one or more layers may comprise polystyrene sheets. In another example, the medium providing a water-equivalent depth of at least 3 mm may comprise material composite sheets.

According to the invention, the component providing a water-equivalent depth of at least 3 mm is preferably different from water.

In particular, the at least one beam profile is used to create a 2D dose distribution, i.e. a plurality of beam profiles forming a 2D dose distribution.

The training of the NN is carried out such that for a given array geometry, the dose at the detector positions is calculated for different beam configurations using Monte Carlo simulations. For the same beam configurations, the corresponding 3D dose distribution in a water phantom is Monte Carlo simulated. In the training, the dose profile from the array is put into relation to the 3D dose distribution.

According to an embodiment, the method further comprises calculating the 3D dose distribution from at least one radiation beam profile in one depth of the profile detector for different beam configurations. This enables fast and efficient LINAC commissioning.

In an embodiment, training the neural network comprises correlating the at least one beam profile with the respective 3D dose distribution in water, in particular wherein the beam profile in water or a water-equivalent material is measured using the profile detector. Thereby, training data are defined for the training of the neural network, in particular for a certain profile detector with known specifications, such as detector arrangements.

In an embodiment, the method further comprises validating the trained neural network, comprising:
- Inputting measured commissioning data to the trained neural network,
- Outputting, by the trained neural network, a corresponding 3D dose distribution,
- Slicing the data of the 3D dose distribution at one or more predetermined depths,
- Comparing the slices of the data with data measured at the one or more predetermined depths.

In a second step, the trained neural network may be validated based on measured LINAC commissioning data as verification input. This allows for the assessment of the model's predictive accuracy by comparing its outputs to real-world measurements.

Furthermore, the validation process can help identify potential errors or inconsistencies in the beam data collection.

Another aspect of the present invention describes a method for calculating a 3D dose distribution in water, in particular for use in radiotherapy, the method comprising:
- Defining a set of irradiation parameters, in particular based on requirements of a treatment planning system of a radiation machine in radiotherapy,
- Recording at least one beam profile, in particular in a water-equivalent depth of at least 3 **mm,** in particular within a medium comprising at least one solid component, for the set of irradiation parameters, in particular using a profile detector,
- Performing a calibration of the profile detector in order to provide the dose in water in Gy,
- Calculating the dose profile of the beam in water using the dose calibration of the profile detector,
- Calculating the 3D dose distribution in water for one or more of the irradiation parameters of the set of irradiation parameters based on the recorded at least one calibrated beam profile.

Gy (gray) may be understood as a unit of ionizing radiation dose in the International System of Units (SI), defined, for instance, as the absorption of one joule of radiation energy per kilogram of matter.

The method allows to perform a full LINAC commissioning without using a water phantom. As such, the invention is intended to provide waterless dose profile determinations whose data quality corresponds to that of water measurements. As an advantage, no complex and lengthy measurements with a water phantom are necessary.

The user may set a field size and depth which is required by the TPS. The invention will calculate the full 3D dose distribution in a water phantom at this field size. From this, all the data required by the TPS is extracted. The 3D dose distribution can also be extracted.

In an embodiment, calculating the 3D dose distribution in water based on the at least one beam profile comprises providing a real geometry of the detector, in particular of the profile detector, and a corresponding geometry in water, wherein the dose distributions of the real geometry of the detector and the geometry in water are calculated, preferably based on Monte Carlo simulations, providing at least one radiation quality specific correction factor. Thereby, the method is tailored to the specific radiological properties of the detector. Thus, different detectors may be used and calibrated for different radiation qualities with the disclosed method, thereby providing the dose in water as output.

In an embodiment, a neural network trained for calculating a 3D dose distribution in water, in particular the neural network describes above, is used for calculating the 3D dose distribution in water. Neural networks can significantly reduce computation time compared to traditional Monte Carlo simulations, which are computationally intensive and time-consuming. Further, NN may predict dose distributions with high accuracy and can effectively process and learn from complex, high-dimensional data such as 3D CT images and PET scans, allowing for more comprehensive dose calculations that consider patient-specific anatomical information. By reducing the computational resources required for dose calculations, neural networks can also make advanced dose estimation techniques more accessible in clinical settings with limited computing power.

In an alternative embodiment, an algorithm is used for calculating the 3D dose distribution in water, wherein the calculation is based on one or more of a beam propagation geometry, profile symmetry and/or depth dose curve.

As an alternative to a trained **NN,** a "classical" algorithm can be used to reconstruct the 3D dose distribution from beam profiles. Here, the beam propagation geometry and profile symmetries are taken into account, as well as the depth dose curve, allowing to derive 2D dose distributions for each depth in water.

In another embodiment, recording the at least one beam profile comprises measuring one or more beam profiles at one or more depths, in particular using the profile detector. Thereby, the accuracy of the method could be improved.

In an embodiment, the method further comprises one or more of:
- Calculating, using a neural network, in particular the neural network trained by the method described above, a large set of beam profiles suitable for commissioning of a linear accelerator for radiotherapy, based on the at least one beam profile, and/or
- Testing the consistency of the calculated beam profiles and 3D dose distributions within the large set of beam profiles, in particular by comparing one or more calculated beam profiles with corresponding measured beam profiles and/or by comparing a predicted profile dataset, predicted by using the neural network describes above, with a corresponding measured profile dataset.

Calculating a large set of beam profiles allows to save time during the LINAC commissioning while maintaining a high data accuracy. Testing the consistency of the calculated beam profiles and 3D dose distribution within the large set of beam profiles assures data accuracy and reliability.

In an embodiment, the 1D, 2D and/or 3D dose distribution in water, water-equivalent material and/or air is calculated using Monte Carlo simulations. Monte Carlo simulations are considered the gold standard for dose calculations in radiotherapy because they model radiation transport at the particle level. They accurately account for complex interactions such as electromagnetic and nuclear processes, scattering effects, and tissue inhomogeneities, ensuring precise dose distribution predictions.

Another aspect of the present discloser relates to a use of the method as described above for commissioning of a beam of a linear accelerator for use in radiotherapy, and/or for validation of a treatment planning system beam model in radiotherapy, and/or for tuning a linear accelerator for use in radiotherapy after a repair or maintenance check.

Another aspect of the present invention describes a detector system, comprising a profile detector, and a computing system configured to perform the methods described above.

The invention, thus, also provides a detector system with an array and specific algorithms allowing to perform a full LINAC commissioning without using a water phantom. As such, the invention provides waterless dose profile determinations whose data quality corresponds to that of water measurements. As an advantage, no complex and lengthy measurements with a water phantom are necessary. Additionally, a profile detector is smaller than a water phantom and can therefore also be used for irradiation systems with measuring rooms that are difficult to access or where space is limited. Another advantage of the system is easier handling in use and less space needed for storage when compared to a water phantom. In addition, as array detectors are more affordable than water phantom scan-systems, the disclosed method provides a cost-effective alternative to perform a LINAC commissioning.

In an embodiment, a predetermined amount of material with water-equivalent or water-like radiological properties providing a water-equivalent total depth of at least 3 **mm,** in particular **a,** preferably solid, material, in particular polystyrene, is placed on top of a top-surface of the profile detector, in particular wherein the material is placed using an automated setup, in particular comprising a robotic arm.

In other words, automated hardware in which depth information for waterless commissioning are provided are realized by placing plates made from water-equivalent material, in particular polystyrene plates or plates made from any other suitable solid material or water-filled plates on top of the detector. A different number of polystyrene plates can be placed on the array manually or in an automated way such as a robotic arm, thereby allowing measurements without interruptions due to human intervention.

In another embodiment, a first side of the array is covered by a first material layer, in particular a polymer layer, preferably a polystyrene layer, with a first layer thickness, and a second side opposite of the first side of the array is covered by a second material layer, in particular a polymer layer, preferably a polystyrene layer, with a second layer thickness, different from the first layer thickness; and wherein the profile detector is rotatable with respect to a radiation source.

In this alternative, the detector is equipped with two material layers of different thicknesses on each side. Thus, placing a stack of material plates on top of the detector is not necessary.

According to an embodiment, the material layer has a wedge-profile. In particular, as an alternative or in addition to the stack of material layers and/or the two-sided material layers on a rotatable detector, a wedge made from water-equivalent or high-Z material can be used to measure a full depth dose curve. Thereby, either no stacking and no rotation of the detector are necessary as the material thickness varies site-specific; or, if used in addition to the above methods, a more comprehensive set of depths can be measured allowing for more accuracy.

Another aspect of the present disclosure relates to a detector system, comprising a profile detector, and a computing system providing a neural network trained by the methods describes above. Thereby, no on-site training is necessary and the system is ready for waterless LINAC commissioning.

According to an embodiment, the computing system is adapted to compute, using the neural network, 3D dose distributions from at least one beam profile measured with the profile detector.

In an embodiment, the computing system is adapted for commissioning of a beam of a linear accelerator for use in radiotherapy, and/or for validation of a treatment planning system beam model in radiotherapy, and/or for tuning a linear accelerator for use in radiotherapy after a repair or maintenance check.

All properties of the methods of the present disclosure also apply to the systems.

### Brief description of the drawings

The features, objects, and advantages of the present disclosure will become more apparent from the detailed description set forth below when taken in conjunction with the drawings in which like reference numerals refer to similar elements.
Figure 1 depicts a flow chart of a method 100 for calculating a 3D dose distribution in water from a beam profile measured;
Figure 2 depicts a flow chart of a method 200 for training a neural network for calculating a 3D dose distribution in water from a beam profile measured;
Figure 3 depicts a block diagram of a detector system 300.

### Detailed description of the figures

The invention will now be described in more detail with reference to an embodiment example, but is not limited to the embodiment example. Further embodiments result from combining the features of individual or several claims with one another and/or with individual or several features of the embodiment example.

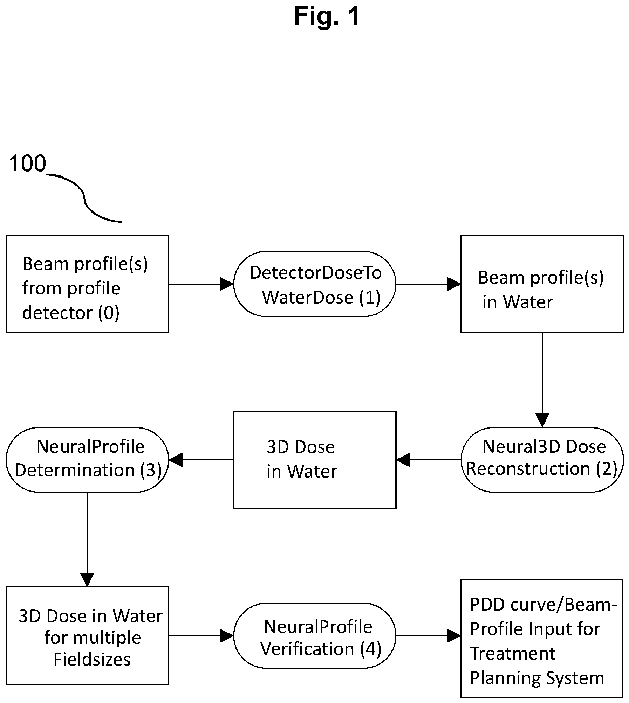

Figure 1 depicts a flow chart of a method 100 for calculating a 3D dose distribution in water from a beam profile measured according to an embodiment.

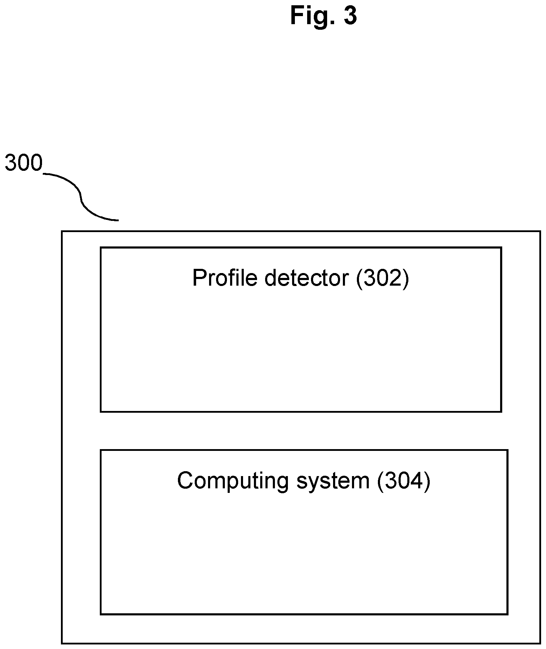

At step 0, at least one beam profile is measured. The beam profile or beam profiles are defined based on a set of irradiation parameters required by a treatment planning system of a radiation machine in radiotherapy. The beam profile(s) are recorded using a profile detector as described in more detail below, with reference to Figure 3.

Such recording of the at least one beam profile comprises measuring one or more beam profiles at one or more depths, in particular using the profile detector.

This is realized by putting water-equivalent material onto the detector or by flipping the detector over, having a different amount of build-up material below and above the detector. Or, instead of placing different amounts of material onto the detector, this is realized by performing the irradiation at 90° gantry: the LINAC gantry can be rotated by 90° such that the profile detector is irradiated from the side. This results in a depth dose curve with one array axis defining the depth coordinate. In said arrangement, the resulting depth profile of the profile detector could be converted into a depth profile in a water phantom by a trained NN.

In step (1) the beam profile, which may be a set of beam profiles assembled to form a 2D dose distribution, is correlated to a beam profile or 2D dose distribution in water. This step can be understood as a calibration of the profile detector to provide the dose in water.

In step (2), a 3D dose distribution in water is calculated for one or more of the irradiation parameters set above based on the recorded and calibrated beam profile(s).

To this end, a real geometry of the profile detector could be provided and a corresponding geometry in water could be calculated by means of a Monte Carlo simulation. The comparison between these two geometries can be used to provide a radiation quality specific correction factor.

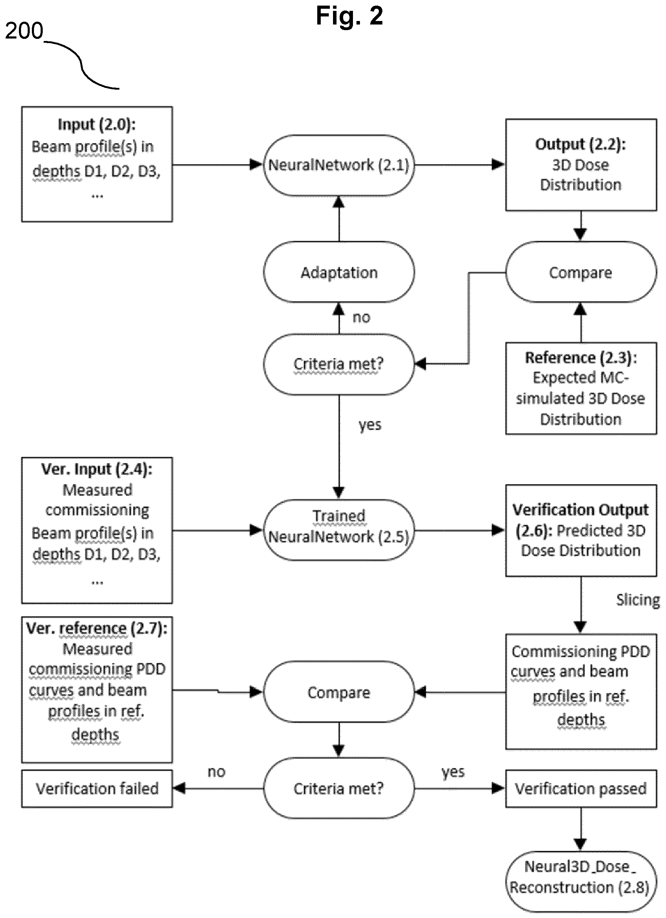

In particular, a neural network trained for calculating a 3D dose distribution in water, as described below with reference to Figure 2, is used for calculating the 3D dose distribution in water.

At step (3), the trained neural network calculates a large set of beam profiles suitable for commissioning of a linear accelerator for radiotherapy, based on the at least one beam profile. For example, this comprises determining 3D dose distributions in water for multiple field sizes.

In particular, a full beam profile commissioning set is derived by sparse measurements using the trained neural network. Thereby, it is possible to input only one beam profile/PDD curve measured at a large field size and to calculate the other beam profiles/PDD curves by the trained NN. The concept of this approach has been established in two publications (DOIs: 10.1016/j.radonc.2020.09.057 and 10.1002/mp.16212). The benefit of the approach is to save time during LINAC commission while maintaining a high data accuracy.

In step (4), the consistency of the calculated beam profiles and 3D dose distributions within the large set of beam profiles is calculated, in particular by comparing one or more calculated beam profiles with corresponding measured beam profiles and/or by comparing a predicted profile dataset, predicted by using the trained neural network, with a corresponding measured profile dataset.

A possible implementation is such that after step 3, one or more (randomly chosen) verification profiles different from the input profiles are measured and compared to the profile predicted by the NN. This can be done by criteria such as gamma passing rates. In addition, a visual inspection can be provided.

Another approach consists of a pretraining step of a NN using golden beam data and/or previous commissioning data. In this scenario, N profiles and PDD curves are measured with N being the number of required quadratic field sizes. For N-1 field sizes as input to the trained NN, the missing profile dataset will be predicted. This can be done for all field sizes. The predicted profile dataset will then be compared to the measured profile dataset, thereby checking the consistency of measurements.

The thereby created full profile set may serve as input for a treatment planning system.

In an example, the use of the method at the user site will be as follows:
For a given radiation quality (e.g. 6 MV FFF), the user measures at least one beam profile for a given set of beam settings. In an example, this is only a set of field sizes. This is done for 1, 2 or 3 depths which are realized by putting water-equivalent material onto the detector or by flipping the detector over, having a different amount of build-up material below and above the detector. Instead of placing different amounts of material onto the detector, an irradiation at 90° gantry could be performed as well.

Next, the user sets the field size and depth which is required by the TPS. The method calculates the full 3D dose distribution in a water phantom at this field size. From this, all the data required by the TPS is extracted. The 3D dose distribution can also be extracted.

These steps can be repeated for all required beam qualities of the radiation machine.

One possible application of the method and example procedure is beam commissioning (which is described above), validation of the TPS beam model, tuning of the LINAC after repair and regular checks which are performed on a monthly, quarterly, or yearly basis.

In an example, the method comprises a software tool that allows users to view the 3D dose distribution in water in real-time during measurement tasks such as LINAC tuning or quality checks.

We note, that a part of commissioning is also reference dosimetry (i.e. measurement of the accurate dose according to a specific protocol). Reference dosimetry can be performed using (i) the calibrated profile detector, or (ii) a calibrated ionization chamber or solid-state detector in a solid-state or water phantom.

In an example, the profiles in a depth chosen by the user and simultaneously the PDD curve are displayed in real time. This can be used for tuning the LINAC, e.g., after repair. Both profiles and PDD curves can be compared to a suited reference.

Furthermore, on the basis of the beam profile measurements with the profile detector, the accelerator's energy can be calculated, as the lateral shape of the profile depends on the energy distribution. If the NN is further trained to perform this conversion, the energy distributions can be efficiently calculated from the beam profiles.

Figure 2 depicts a flow chart of a method 200 for training and validating a neural network for calculating a 3D dose distribution in water from measured at least one beam profile, according to an embodiment.

In step **2.1,** a neural network is trained to map an input 2D dose distribution (step 2.0) onto a 3D dose distribution as output (step 2.2) using the MC-simulated expected 3D dose distribution as reference (step 2.3).

In more detail, in step 2.0 at least one beam profile is determined, in particular for predetermined irradiation parameters, and in particular using the profile detector as described below with reference to Figure 3.

In step 2.3, a 3D dose distribution in water is calculated, in particular for the predetermined irradiation parameters and based on the recorded at least one beam profile, using a Monte Carlo simulation.

The neural network is trained at step 2.1 for calculating the 3D dose distribution in water using the determined at least one beam profile (2.0) and the calculated 3D dose distribution in water (2.3).

The training of the neural network comprises correlating the at least one beam profile with the respective 3D dose distribution in water. If the neural network outputs at step 2.2 a 3D dose distribution which compares to the 3D dose distribution calculated by the Monte Carlo simulation within predetermined criteria, the neural network is trained and ready for use and/or for validation at step 2.5. If the comparison does not meet the predetermined criteria, the neural network is iteratively adapted.

The method further comprises validating the trained neural network at steps 2.4-2.8.

In step 2.4, the trained neural network is provided with measured LINAC commissioning data as verification input.

The trained NN then predicts a 3D dose distribution at step 2.5 and outputs the 3D dose distribution at step 2.6.

By slicing, the PDD curves and beam profiles in reference depths are derived from the 3D dose distribution.

The PDD curves and beam profiles are compared to the verification reference data in step 2.7 by using metrics such as gamma rates.

If the passing criteria of the metric are met for a representative, predetermined number of verification data, the NN passes the verification process at step 2.8 and can be integrated into applications.

Figure 3 depicts a block diagram of a detector system 300.

The detector system 300 comprises a profile detector 302, and a computing system 304 configured to perform the methods above. In a preferred example, the system is equipped with a computing system providing a neural network trained by the methods described above and is adapted to compute, using the neural network, 3D dose distributions from at least one beam profile measured with the profile detector.

The computing system is adapted for commissioning of a beam of a linear accelerator for use in radiotherapy, and/or for validation of a treatment planning system beam model in radiotherapy, and/or for tuning a linear accelerator for use in radiotherapy after a repair or maintenance check.

In an example, the detector array is equipped with a predetermined number of material with water-equivalent or water-like radiological properties, in particular polystyrene. Sheets of this material are placed on top of a top-surface of the profile detector. The placement of the material can be performed either manually or by using an automated setup, in particular comprising a robotic arm. Using automation, the change of simulated irradiation depths (resembled by the number of material sheets or plates) can be performed in a closed environment without interrupting a measurement by human intervention.

In another example, a first side of the profile detector is covered by a first material layer, in particular a polymer layer, preferably a polystyrene layer, with a first layer thickness, and a second side opposite of the first side of the detector array is covered by a second material layer, in particular a polymer layer, preferably a polystyrene layer, with a second layer thickness, different from the first layer thickness. In this example, the profile detector is rotatable with respect to a radiation source. By rotating the array, different depth profiles can be recorded.

Herein, first and second material layers are preferably from same material, but may also be from different materials. Preferably, the material is a polymer, but the material may also be a metal, such as aluminum, or other material with suitable radiological properties, in particular water-filled layers with a surrounding solid outer material.

In an example, the material layer has a wedge-profile. The wedge is in particular made from a metal or a water-equivalent, solid material such as polymer, in particular polystyrene and is used to measure a full depth dose curve.

In another example, the LINAC gantry can be rotated by 90° such that the profile detector is irradiated from the side. This results in a depth dose curve with one array axis defining the depth coordinate. The resulting depth profile of the profile detector is converted into a depth profile in a water phantom by a trained NN.

### Reference signs

- 100: Method for calculating a 3D dose distribution in water from a beam profile measured
- 0-4: Steps of method 100
- 200: Method for training a neural network for calculating a 3D dose distribution in water from a beam profile measured
- 2.0-2.8: Steps of method 200
- 300: Detector system
- 302: Profile detector
- 304: Computing system

## Claims

1. **Method for training** (200) a neural network for calculating a 3D dose distribution in water, in particular for use in radiotherapy, the method comprising:
- Determining at least one beam profile, in particular in a water-equivalent depth of at least 3 mm, in particular within a medium comprising at least one solid component, in particular for predetermined irradiation parameters, in particular using a profile detector (302),
- Calculating a 3D dose distribution in water, in particular for the predetermined irradiation parameters, based on the recorded at least one beam profile,
- Training a neural network for calculating the 3D dose distribution in water using the determined at least one beam profile and the calculated 3D dose distribution in water.

2. The method (200) of claim 1, further comprising:
- Calculating the 3D dose distribution from at least one beam profile of the profile detector for different beam configurations.

3. The method (200) of any preceding claim, wherein training the neural network comprises correlating the at least one beam profile with the respective 3D dose distribution in water, in particular wherein the beam profile is measured using the profile detector.

4. The method (200) of any preceding claim, further comprising validating the trained neural network, comprising:
- Inputting measured commissioning data to the trained neural network,
- Outputting, by the trained neural network, a corresponding 3D dose distribution,
- Slicing the data of the 3D dose distribution at one or more predetermined depths,
- Comparing the slices of the data with data measured at the one or more predetermined depths.

5. **Method for calculating** (100) a 3D dose distribution in water, in particular for use in radiotherapy, the method comprising:
- Defining a set of irradiation parameters, in particular based on requirements of a treatment planning system of a radiation machine in radiotherapy,
- Recording at least one beam profile, in particular in a water-equivalent depth of at least 3 mm, in particular within a medium comprising at least one solid component, for the set of irradiation parameters, in particular using a profile detector (302),
- Performing a calibration of the profile detector (302) in order to provide the dose in water in Gy, alternatively using at least one calibrated radiation detector to provide the dose in water in Gy,
- Calculating the dose profile of the beam in water using the dose calibration of the profile detector (302),
- Calculating the 3D dose distribution in water for one or more of the irradiation parameters of the set of irradiation parameters based on the recorded at least one calibrated beam profile.

6. The method (100, 200) of any preceding claim, wherein calculating the 3D dose distribution in water based on the at least one beam profile comprises:
- Providing a real geometry of the detector, in particular of the profile detector (302), and a corresponding geometry in water, wherein the dose distributions of the real geometry of the detector and the geometry in water are calculated, preferably based on Monte Carlo simulations, and
- Providing at least one radiation quality specific correction factor.

7. The method (100) of any preceding claim, wherein a neural network trained for calculating a 3D dose distribution in water, in particular the neural network of any of claims **1-**4, is used for calculating the 3D dose distribution in water.

8. The method (100) of any preceding claim, wherein an algorithm is used for calculating the 3D dose distribution in water, wherein the calculation is based on one or more of a beam propagation geometry, profile symmetry and/or depth dose curve.

9. The method (100, 200) of any preceding claim, wherein recording the at least one beam profile comprises measuring one or more beam profiles at one or more depths, in particular using the profile detector.

10. The method (100) of any preceding claim, further comprising one or more of:
- Calculating, using a neural network, in particular the neural network trained by the method of claim **1,** a large set of beam profiles suitable for commissioning of a linear accelerator for radiotherapy, based on the at least one beam profile, and/or
- Testing the consistency of the calculated beam profiles and 3D dose distributions within the large set of beam profiles, in particular by comparing one or more calculated beam profiles with corresponding measured beam profiles and/or by comparing a predicted profile dataset, predicted by using the neural network of claim **1,** with a corresponding measured profile dataset.

11. The method (100, 200) of any preceding claim, wherein the 1D, 2D and/or 3D dose distribution in water, water-equivalent material and/or air is calculated using Monte Carlo simulations.

12. **Use of the method** (100) of any preceding claim for commissioning of a beam quality of a linear accelerator for use in radiotherapy, and/or for validation of a treatment planning system beam model in radiotherapy, and/or for tuning a linear accelerator for use in radiotherapy after a repair or maintenance check.

13. **Detector system (300),** comprising:
- A profile detector (302), and
- A computing system (304) configured to perform the method (100, 200) of any of claims 1 - 11.

14. The detector system (300) of claim 13, wherein a predetermined amount of material with water-equivalent or water-like radiological properties, in particular a, preferably solid, material, in particular polystyrene, is placed on top of a top-surface of the profile detector, providing a total water-equivalent depth of at least 3 mm, in particular, wherein the material is placed using an automated setup, in particular comprising a robotic arm.

15. The detector system (300) of claim 13, wherein a first side of the array is covered by a first material layer, in particular a polymer layer, preferably a polystyrene layer, with a first layer thickness, and a second side opposite of the first side of the array is covered by a second material layer, in particular a polymer layer, preferably a polystyrene layer, with a second layer thickness, different from the first layer thickness; and wherein the profile detector (302) is rotatable with respect to a radiation source.

16. The detector system (300) of any of claims 13-15, wherein at least one of the material layers has a wedge-profile.

17. Detector system (300), comprising:
- A profile detector (302), and
- A computing system providing a neural network trained by the method of any of claims 1 - 11.

18. The detector system of any of claims 13-17, wherein the computing system is adapted to compute, using the neural network, 3D dose distribution from at least one beam profile measured with the profile detector (302).

19. The detector system of any of claims 13-18, wherein the computing system (304) is adapted for commissioning of a beam of a linear accelerator for use in radiotherapy, and/or for validation of a treatment planning system beam model in radiotherapy, and/or for tuning a linear accelerator for use in radiotherapy after a repair or maintenance check.

## Amended claims

### Amended claims in accordance with Rule 137(2) EPC.

1. **Method for training** (200) a neural network for calculating a 3D dose distribution in water, in particular for use in radiotherapy, the method comprising:
- Recording at least one beam profile, in a water-equivalent depth of at least 3 mm within a medium comprising at least one solid component, for given irradiation parameters using a profile detector (302),
- Calculating a 3D dose distribution in water, for the given irradiation parameters, based on the recorded at least one beam profile,
- Training a neural network for calculating the 3D dose distribution in water using the recorded at least one beam profile and the calculated 3D dose distribution in water.

2. The method (200) of claim 1, further comprising:
- Calculating the 3D dose distribution from at least one beam profile of the profile detector (302) for different beam configurations.

3. The method (200) of any preceding claim, wherein training the neural network comprises correlating the at least one beam profile with the respective 3D dose distribution in water.

4. The method (200) of any preceding claim, further comprising validating the trained neural network, comprising:
- Inputting measured commissioning data to the trained neural network,
- Outputting, by the trained neural network, a corresponding 3D dose distribution,
- Slicing the data of the 3D dose distribution at one or more predetermined depths,
- Comparing the slices of the data with data measured at the one or more predetermined depths.

5. **Method for calculating** (100) a 3D dose distribution in water, in particular for use in radiotherapy, the method comprising:
- Defining a set of irradiation parameters, in particular based on requirements of a treatment planning system of a radiation machine in radiotherapy,
- Recording at least one beam profile in a water-equivalent depth of at least 3 mm within a medium comprising at least one solid component, for the set of irradiation parameters using a profile detector (302),
- Performing a calibration of the profile detector (302) in order to provide the dose in water in Gy, alternatively using at least one calibrated radiation detector to provide the dose in water in Gy,
- Calculating the dose profile of the beam in water using the dose calibration of the profile detector (302),
- Calculating the 3D dose distribution in water for one or more of the irradiation parameters of the set of irradiation parameters based on the recorded at least one calibrated beam profile.

6. The method (100, 200) of any preceding claim, wherein calculating the 3D dose distribution in water based on the at least one beam profile comprises:
- Providing a real geometry of the profile detector (302), and a corresponding geometry in water, wherein the dose distributions of the real geometry of the profile detector (302) and the geometry in water are calculated, preferably based on Monte Carlo simulations, and
- Providing at least one radiation quality specific correction factor.

7. The method (100) of any preceding claim, wherein a neural network, trained according to any of claims 1-4 for calculating a 3D dose distribution in water, is used for calculating the 3D dose distribution in water.

8. The method (100) of any of claims 1 - 6, wherein an algorithm is used for calculating the 3D dose distribution in water, wherein the calculation is based on one or more of a beam propagation geometry, profile symmetry and/or depth dose curve.

9. The method (100, 200) of any preceding claim, wherein recording the at least one beam profile comprises measuring one or more beam profiles at one or more depths using the profile detector (203).

10. The method (100) of any preceding claim, further comprising one or more of:
- Calculating, using a neural network trained by the method of claim 1, a large set of beam profiles suitable for commissioning of a linear accelerator for radiotherapy, based on the at least one beam profile, and/or
- Testing the consistency of the calculated beam profiles and 3D dose distributions within the large set of beam profiles, in particular by comparing one or more calculated beam profiles with corresponding measured beam profiles and/or by comparing a predicted profile dataset, predicted by using the neural network of claim 1, with a corresponding measured profile dataset.

11. The method (100, 200) of any preceding claim, wherein the 1D, 2D and/or 3D dose distribution in water, water-equivalent material and/or air is calculated using Monte Carlo simulations.

12. **Use of the method** (100) of any preceding claim for commissioning of a beam quality of a linear accelerator for use in radiotherapy, and/or for validation of a treatment planning system beam model in radiotherapy, and/or for tuning a linear accelerator for use in radiotherapy after a repair or maintenance check.

13. **Detector system (300),** comprising:
- A profile detector (302), and
- A computing system (304) configured to perform the method (100, 200) of any of claims 1 - 11.

14. The detector system (300) of claim 13, wherein a predetermined amount of material with water-equivalent or water-like radiological properties, in particular a, preferably solid, material, in particular polystyrene, is placed on top of a top-surface of the profile detector, providing a total water-equivalent depth of at least 3 mm, in particular, wherein the material is placed using an automated setup, in particular comprising a robotic arm.

15. The detector system (300) of claim 13, wherein a first side of the array is covered by a first material layer, in particular a polymer layer, preferably a polystyrene layer, with a first layer thickness, and a second side opposite of the first side of the array is covered by a second material layer, in particular a polymer layer, preferably a polystyrene layer, with a second layer thickness, different from the first layer thickness; and wherein the profile detector (302) is rotatable with respect to a radiation source.

16. The detector system (300) of any of claims 13-15, wherein at least one of the material layers has a wedge-profile.

17. The detector system (300) of any of claims 13-16 further comprising:
- A neural network trained by the method of any of claims 1 - 4.

18. The detector system of claim 17, wherein the computing system is adapted to compute, using the neural network, 3D dose distribution from at least one beam profile measured with the profile detector (302).

19. The detector system of any of claims 13-18, wherein the computing system (304) is adapted for commissioning of a beam of a linear accelerator for use in radiotherapy, and/or for validation of a treatment planning system beam model in radiotherapy, and/or for tuning a linear accelerator for use in radiotherapy after a repair or maintenance check.
